# FASCICULE DE BREVET EUROPEEN

(11) **EP 2 890 329 B1**
(45) Date de publication et mention de la délivrance du brevet: **10.10.2018**
(21) Numéro de dépôt: 13774713.5
(22) Date de dépôt: 28.08.2013
(51) Int. Cl.: A61D 17/00

(54) **DISPOSITIF DE DETECTION ET DE SIGNALISATION DE LA PHASE PREPARATOIRE DE MISE-BAS D'UN BOVIN OU D'UN EQUIDE, PROCEDE ET PRODUIT PROGRAMME D'ORDINATEUR CORRESPONDANTS**
VORRICHTUNG ZUR DETEKTION UND ANZEIGE DER VORBEREITUNGSPHASE DER GEBURT EINES RINDER- ODER PFERDEARTIGEN TIERES SOWIE ZUGEHÖRIGES VERFAHREN UND COMPUTERPROGRAMMPRODUKT
DEVICE FOR DETECTING AND INDICATING THE PREPARATORY PHASE OF BIRTHING A BOVINE OR EQUINE ANIMAL, AND CORRESPONDING METHOD AND COMPUTER PROGRAM PRODUCT

(30) Priorité: 31.08.2012 FR 1258128; 11.09.2012 FR 1258539
(43) Date de publication de la demande: 08.07.2015
(73) Titulaire: UNION EVOLUTION, 35700 Rennes (FR)
(72) Inventeur: PHILIPOT, Jean-Michel, F-35830 Betton (FR)
(74) Mandataire: Ermeneux, Bertrand
(86) Numéro de dépôt international: PCT/FR2013/051982
(87) Numéro de publication internationale: WO 2014/033403

(56) Documents cités:
- WO-A1-2007/119070
- FI-A- 990 112
- FR-A1- 2 392 599
- FR-A1- 2 618 051
- FR-A1- 2 689 754
- JP-A- 2011 234 668
- US-A1- 2010 036 277
- US-A1- 2010 331 739

## Description

### 1. Domaine de l'invention

Le domaine de l'invention est celui de l'agriculture, et en particulier de la surveillance des bovins et des équidés.

Plus précisément, l'invention concerne un dispositif et un procédé correspondant de détection et de signalisation de la phase préparatoire de mise-bas d'un bovin ou d'un équidé.

### 2. Etat de la technique

Il est bien connu que, sans intervention humaine, une partie des vaches ou des juments perdent leur nouveau-né à l'occasion de la mise-bas.

Il est donc nécessaire pour un éleveur d'exercer, de jour et de nuit, une surveillance des animaux arrivés à terme, afin de pouvoir intervenir si besoin lors de la parturition pour tenter de sauver le nouveau-né ou de réduire les complications post-partum.

Il est par ailleurs courant d'effectuer plusieurs saillies simultanément au sein d'un même cheptel, ce qui complique la tâche de surveillance de l'éleveur dans la mesure où les vêlages ou les poulinages vont se produire sur une même période.

Afin d'éviter à un éleveur de devoir rester mobilisé en permanence aussi bien de jour que de nuit, et pour lui permettre de se reposer lors de la période de vêlage ou de poulinage, on a proposé différentes techniques.

On connait des techniques de détection de vêlage consistant à introduire des capteurs mesurant la température, la pression et/ou l'acidité à l'intérieur de l'appareil génital d'une vache afin d'identifier l'imminence de la mise-bas. Une de ces techniques consiste par exemple à introduire un thermomètre dans le canal vaginal de l'animal qui sera expulsé avec la poche des eaux.

Un inconvénient de ces techniques intrusives est que l'introduction et le positionnement des capteurs dans l'appareil génital de la vache est délicat, et qu'il est une source d'infection et/ou d'irritation pour ce dernier.

Un autre inconvénient de ces techniques intrusives est que des modifications métaboliques normales de l'animal dues à son activité peuvent entraîner le déclenchement de fausses alertes.

Encore un inconvénient de ces techniques intrusives est qu'elles sont couteuses à mettre en oeuvre.

On a également proposé de fixer un inclinomètre à mercure ou à bille sur la queue d'une vache ou d'une jument, qui va déclencher une alarme à l'approche de la mise-bas, lorsque la queue de l'animal reste relevée.

Un inconvénient de cette technique est qu'elle pose des problèmes de fiabilité. En effet, on constate que des alertes peuvent se déclencher de façon intempestives, par exemple lorsque l'animal change de position ou se couche.

On a également proposé de sangler la taille d'une vache avec une ceinture qui mesure l'activité utérine de l'animale et émet une alarme lorsque la fréquence des contractions augmente.

Cette technique présente plusieurs inconvénients : la ceinture présente un danger pour l'animal si elle est trop serrée. Elle ne doit cependant pas être trop lâche au risque de ne pas fonctionner correctement, ce qui impose de la resserrer toutes les 3 à 4 heures. En outre cette technique est onéreuse et ne permet pas dans certains cas de distinguer les déformations dues au travail de l'animal d'autres déformations résultant d'une activité normale de l'animal pour manger ou se déplacer.

Les documents JP2011-234668, WO2007/119070 et US2010/0036277 constituent l'état de la technique pertinent de la présente invention. Ils divulguent des dispositifs de détection et de signalisation de la phase préparatoire de mise-bas d'un animal étant adaptés à être fixés sur la queue de l'animal.

### 3. Objectifs de l'invention

L'invention a notamment pour objectif de pallier les inconvénients de l'état de la technique cités ci-dessus.

Plus précisément l'invention a pour objectif de fournir une technique de détection et de signalement de la phase préparatoire de mise-bas d'un bovin ou d'un équidé qui soit fiable.

L'invention a notamment pour objectif, dans au moins un mode de réalisation particulier de l'invention, de proposer une telle technique de détection et de signalement qui présente un taux de détection de la phase préparatoire de mise-bas supérieur à 80%

Un autre objectif de l'invention est de fournir une telle technique de détection et de signalement qui soit simple à mettre en oeuvre et d'un coût de revient réduit.

Un objectif de l'invention est également de proposer une telle technique de détection et de signalement qui soit réutilisable.

Encore un objectif de l'invention est de fournir une technique de détection et de signalement de la phase préparatoire de mise-bas d'un bovin ou d'un équidé qui nécessite peu de maintenance et qui soit autonome en énergie sur un période de plusieurs années.

### 4. Exposé de l'invention

Ces objectifs, ainsi que d'autres qui apparaitront par la suite sont atteints à l'aide d'un dispositif de détection et de signalisation de la phase préparatoire de mise-bas d'un bovin ou d'un équidé, apte à être fixé sur la queue dudit bovin ou dudit équidé.

Il convient de noter à titre liminaire que de manière générale la pose d'un dispositif sur la queue de l'animal est une opération aisée, ce qui constitue un avantage du dispositif de détection et de signalisation selon l'invention.

Dans le cadre de l'invention, on entend l'expression "phase préparatoire de mise-bas" dans son acception générale. Il s'agit ainsi de la phase précédant l'engagement du foetus dans le bassin, d'une durée généralement comprise entre 2 et 24 heures, au cours de laquelle se produisent notamment les premières contractions utérines et la dilatation du col de l'utérus.

Selon l'invention un tel dispositif de détection et de signalisation comprend :
- des moyens de mesure des composantes de l'accélération dudit dispositif selon au moins un axe, et préférentiellement selon trois axes orthogonaux ;
- des moyens de traitement des mesures desdites composantes, destinés à détecter une occurrence de ladite phase préparatoire, comprenant des moyens de comparaison d'une séquence desdites mesures avec deux, trois ou quatre séquences de données de référence représentatives chacune d'une manifestation physiologique ou comportementale différente dudit bovin ou dudit équidé appartenant au groupe comprenant :
- contractions musculaires de l'utérus ;
- coliques résultant de douleurs utérines ;
- levée de la queue ;
- suractivité ;
- des moyens de génération d'un signal de radiocommunication configurés de sorte à générer ledit signal dans le cas où ladite occurrence est détectée ;
- des moyens d'émission dudit signal généré à destination d'un collecteur formant relai ou d'un terminal.

Ainsi, de façon inédite, l'invention propose d'exploiter les mesures de l'accélération de la queue d'une vache, d'une jument ou d'une ânesse pour détecter le commencement de la parturition.

Le dispositif selon l'invention présente astucieusement des moyens de traitement des mesures intégrés, ce qui permet de limiter sa consommation d'énergie du fait que la quantité de signaux qu'il émet vers l'extérieur est très réduite et lui confère ainsi une autonomie en énergie particulièrement étendue.

Selon un aspect particulier de l'invention, ledit signal de radiocommunication est un signal numérique.

Il n'est ainsi pas nécessaire de convertir ce signal par la suite.

Dans un mode de réalisation particulièrement avantageux de l'invention, un identifiant dudit dispositif est encapsulé dans ledit signal numérique.

De cette façon, l'éleveur peut être averti de l'identité de l'animal dont la mise-bas est imminente.

Ainsi, en corrélant les mesures des composants de l'accélération en fonction d'une ou plusieurs manifestations physiologiques ou comportementales caractéristiques de la phase préparatoire de mise-bas, il est possible de détecter de façon fine l'entrée dans cette phase et d'écarter les mesures qui ne présentent pas de lien avec des manifestations physiologiques ou comportementales résultant de la mise-bas.

Les inventeurs ont en effet observé que chez l'animal les contractions utérines, les coliques, les levées de queue au cours desquelles la queue reste relevée pendant plusieurs secondes et la suractivité constituaient les manifestations les plus caractéristiques pouvant être observées au travers des mouvements de la queue de l'animal pendant la phase préparatoire de mise-bas et que chacune de ces manifestations déclenchait une séquence spécifique et reproductible de mouvements au moins sur une partie d'une population bovine ou équine. Les inventeurs ont également constaté que ces séquences spécifiques pouvaient varier entre deux races de vaches ou de juments.

Ainsi par exemple, lors du traitement il peut être prévu de comparer la séquence de mesure à une séquence de référence représentative des contractions utérines et à une séquence de référence représentative des coliques résultant de douleurs utérines ou à une séquence de référence représentative des contractions utérines, à une séquence de référence représentative de la levée de queue et à une séquence de référence représentative d'une suractivité.

Selon un aspect préférentiel de l'invention, ladite séquence de données de référence présente, au moins pour une de ses composantes, au moins 3 pics, préférentiellement de 5 à 10 pics, espacés d'une durée de 3 à 4 secondes.

Les inventeurs ont en effet constaté qu'une telle séquence de données était sensiblement universelle chez tous les animaux.

De façon avantageuse, lesdits moyens de traitement comprennent des moyens de détermination des coefficients de corrélation entre ladite séquence de mesures et lesdites séquences de référence.

On peut ainsi quantifier d'une façon simple les différences entre la séquence de mesure et la ou les séquences de référence.

Dans au moins un mode de réalisation de l'invention, lesdits moyens de traitement comprennent des moyens de comparaison d'un produit desdits coefficients de corrélation affectés d'une pondération avec une valeur seuil prédéterminée, et des moyens de génération d'une données d'alerte signifiant qu'une occurrence de ladite phase préparatoire a été détectée lorsque le résultat de ladite comparaison indique que ledit produit desdits coefficients de corrélation affectés d'une pondération est supérieur ou égale à ladite valeur seuil prédéterminée.

Selon un mode de réalisation particulier de l'invention, la pondération de chacun desdits coefficients de corrélation dans ledit produit est égale à 1.

La sensibilité de la détection à chacune des manifestations physiologiques ou comportementales est alors identique.

Préférentiellement, un dispositif de détection et de signalisation tel que ceux décrits ci-dessus comprend un dispositif d'alimentation en énergie électrique autonome desdits moyens de mesure et/ou desdits moyens de traitement et/ou desdits moyens de génération d'un signal et/ou desdits moyens d'émission.

On obtient ainsi un dispositif autonome en énergie.

De façon préférentielle, un dispositif de détection et de signalisation tel que ceux décrits ci-dessus comprend un boitier présentant une portion inférieure configurée pour épouser la forme de ladite queue.

Ainsi, on assure une fixation convenable du dispositif sur la queue.

Cette portion inférieure du boitier peut notamment présenter une forme sensiblement tronconique.

De façon avantageuse, ledit signal émis par lesdits moyens d'émission a une portée comprise entre 150 et 500 mètres.

Ainsi, le signal peut être transmis jusqu'à un relai situé dans, ou à proximité de, l'étable ou la stabule où est parqué l'animal, quel que soit l'emplacement de ce dernier dans le bâtiment. Le signal peut encore être transmis directement sur un terminal de communication de l'éleveur, tel qu'un téléphone portable ou un assistant personnel par exemple, si celui-ci se trouve à proximité du bâtiment.

L'invention concerne également un procédé de détection de la phase préparatoire de mise-bas d'un bovin ou d'un équidé.

Selon l'invention, un tel procédé comprend les étapes suivantes :
- mesure de données représentatives des composantes de l'accélération d'une portion de la queue dudit bovin ou dudit équidé selon au moins un axe, et préférentiellement selon trois axes orthogonaux ;
- traitement des mesures desdites composantes destiné à détecter une occurrence de ladite phase préparatoire, comprenant une étape de comparaison d'une séquence desdites mesures avec deux, trois ou quatre séquences de données de référence représentatives chacune d'une manifestation physiologique ou comportementale dudit bovin ou dudit équidé appartenant au groupe comprenant :
   - contractions musculaires de l'utérus ;
   - coliques résultant de douleurs utérines ;
   - levée de la queue ;
   - suractivité.

De façon avantageuse, ladite étape de traitement comprend :
- une étape de détermination des coefficients de corrélation entre ladite séquence de mesures et lesdites séquences de référence ;
- une étape de comparaison d'un produit desdits coefficients de corrélation affectés d'une pondération avec une valeur seuil prédéterminée, de façon à pouvoir générer un signal d'alerte, destiné à avertir qu'une occurrence de ladite phase préparatoire a été détectée, lorsque le résultat de ladite comparaison indique que ledit produit desdits coefficients de corrélation affectés d'une pondération est supérieur ou égale à ladite valeur seuil prédéterminée

L'invention concerne encore un produit programme d'ordinateur téléchargeable depuis un réseau de communication et/ou enregistré sur un support lisible par ordinateur et/ou exécutable par un processeur, comprenant des instructions de code de programme pour la mise en oeuvre de l'étape de traitement des mesures du procédé de détection décrit ci-dessus, lorsque ledit programme est exécuté par le dispositif décrit ci-dessus.

### 5. Liste des figures

D'autres caractéristiques et avantages de l'invention apparaitront plus clairement à la lecture de la description suivante d'un mode de réalisation de l'invention, donné à titre de simple exemple illustratif et non limitatif, et des dessins annexés parmi lesquels :
- la figure 1 est une vue schématique d'un dispositif selon l'invention fixé sur la queue d'une vache ;
- la figure 2 est une vue de détail du dispositif présenté en référence à la figure 1 ;
- la figure 3 est une illustration des variations de la composante de accélération selon la direction verticale lors d'une séquence typique de contractions utérines ;
- la figure 4 est une illustration sous forme de graphique, et au cours du temps, d'un exemple de résultat du traitement de mesures réalisé au sein du dispositif présenté en référence à la figure 1.

### 6. Description détaillée d'un exemple de mode de réalisation de l'invention

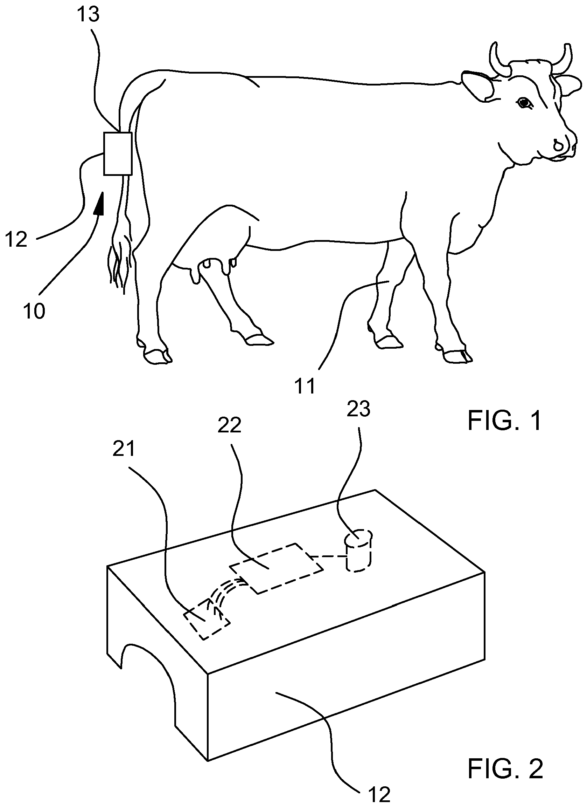

Un dispositif 10 de détection et de signalisation selon l'invention est représenté sur la figure 1, fixé à la queue d'une vache 11.

Le dispositif se présente sous la forme d'un boitier étanche 12 dont la face inférieure 13 est de forme sensiblement tronconique de façon à épouser la forme de la queue de la vache 11.

Pour fixer le boitier 12, on a avantageusement appliqué une substance adhésive, et préférentiellement de la colle utilisée pour fixer les vignettes de type Kamar(marque déposée), sur la queue de l'animal, puis pressé la face inférieure du boitier contre cette surface de colle et enfin entouré ensemble le boitier et la queue de l'animal d'une couche de ruban adhésif.

Comme on peut le voir sur la figure 2, qui est une vue de détail du dispositif 10, le boitier 12 renferme un accéléromètre tridimensionnel 21 de type micro-système électromécanique relié à une carte électronique 22 équipée d'un microcontrôleur MSP 430, qui réalise le traitement des mesures fournies par l'accéléromètre 21, et d'un circuit intégré assurant une optimisation de l'énergie consommée par le boitier.

À l'intérieur du boitier 12, on trouve également une antenne 23 permettant d'émettre un signal numérique conforme à la norme GSM à destination d'un relai ou d'un téléphone portable et une pile L5, qui alimente l'accéléromètre, la carte 22 et l'antenne 23 en énergie.

Le microcontrôleur équipant la carte 22 traite des séquences de mesures délivrées par l'accéléromètre 21 et permet de générer un signal d'alerte, lorsque la phase préparatoire de mise bas est détectée, qui est émis par l'antenne 23.

Dans ce mode de réalisation de l'invention, des séquences constituées de 3 groupes de 64 points de mesures échantillonnés à 16Hz correspondant chacun aux variations d'une composante de l'accélération du boitier 12 pendant une période de 4 secondes, sont traitées en continue par le microcontrôleur.

Ces séquences de mesures sont stockées au fur et à mesure sur une mémoire vive de la carte 22 et ne sont pas conservées après traitement. En effet, le dispositif de traitement étant prévu pour pouvoir fonctionner sans discontinuer plusieurs années, et plus précisément de 5 à 10 ans dans ce mode de réalisation particulier de l'invention, prévoir une mémoire de stockage de capacité suffisante n'est pas envisageable.

Les coefficients de corrélation de chacune de ces séquences de mesures des composantes de l'accélération du boitier selon 3 axes avec quatre séquences de données de référence prédéterminées représentatives respectivement des contractions musculaires de l'utérus, des coliques résultant de douleurs utérines, d'une levée de la queue ou d'une suractivité sont successivement évalués par le microcontrôleur.

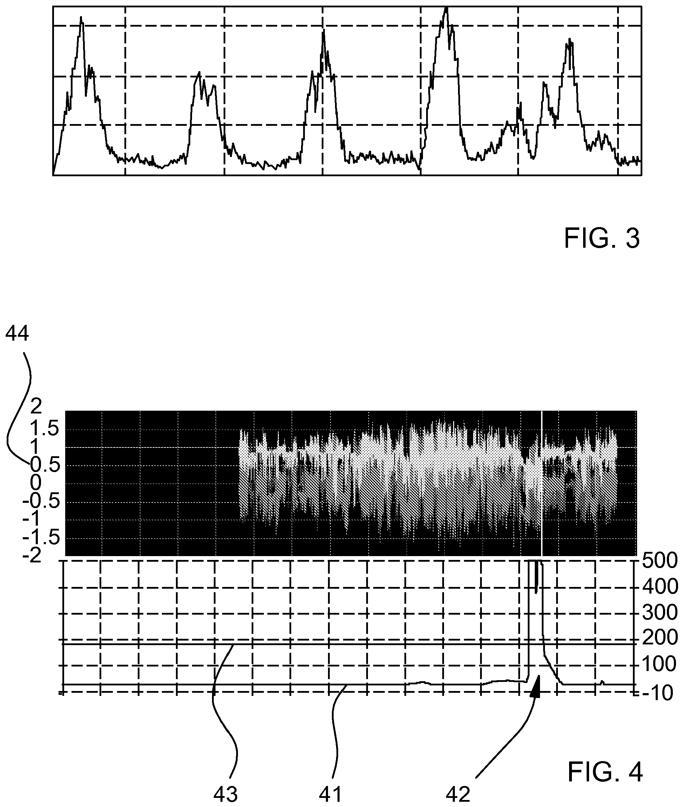

A titre d'illustration, on a représenté sur la figure 3 une séquence de référence correspondant aux variations de la composante de l'accélération selon la direction verticale lorsque se produisent des contractions musculaires de l'utérus.

Dans le cas où un des coefficients de corrélation s'avère inférieure à une valeur minimale prédéterminée propre à ce coefficient, on lui attribue ladite valeur minimale.

Le microcontrôleur affecte ensuite une pondération prédéterminée à chacun des coefficients de corrélation, puis multiplie les coefficients pondérés entre eux, ce qui fournit un indice global représentatif de l'état de l'animal.

Ainsi, par exemple, dans ce mode de réalisation de l'invention, on affecte une pondération respectivement de 3 et de 1,6 aux coefficients de corrélation relatifs aux contractions utérines et à la levée de queue et une valeur de 1 aux coefficients de corrélation relatifs aux coliques résultant de douleurs utérines et à la suractivité, afin d'augmenter la sensibilité de la détection et de pouvoir garantir un taux de détection de l'entrée en phase préparatoire supérieur ou égal à 80%.

Dans des variantes de ce mode de réalisation de l'invention, il peut également être prévu de ne considérer qu'un, deux ou trois coefficients de corrélation pour déterminer l'indice global représentatif de l'état de l'animal et donc de ne prendre en compte qu'une, deux ou trois manifestations physiologiques ou comportementales en vu de détecter l'entrée dans la phase préparatoire de mise-bas.

L'indice global représentatif de l'état de l'animal est finalement comparé à une valeur seuil prédéterminée. Dans le cas où la valeur de l'indice dépasse cette valeur seuil prédéterminée, un signal d'alerte destiné à avertir l'éleveur qu'une occurrence de ladite phase préparatoire a été détectée est généré par le microcontrôleur et émis par l'antenne 23.

Les variations de l'indice global 41 en fonction du temps sur une période précédent une phase préparatoire de mise-bas jusqu'à la détection, signalée par un pic important 42 sont illustrées sur la figure 4. Le seuil prédéterminé est représenté par la ligne horizontale 43 sur cette figure 4.

Dans le cadre supérieur 44 de la figure 4 sont représentées les variations en fonction du temps des composantes selon 3 axes orthogonaux X, Y et Z de l'accélération du dispositif 10 mesurées par l'accéléromètre 21.

## Revendications

1. Dispositif de détection et de signalisation de la phase préparatoire de mise-bas d'un bovin ou d'un équidé, apte à être fixé sur la queue dudit bovin ou dudit équidé, comprenant :
- des moyens de mesure des composantes de l'accélération dudit dispositif selon au moins un axe, et préférentiellement selon trois axes orthogonaux ;
- des moyens de traitement des mesures desdites composantes, destinés à détecter une occurrence de ladite phase préparatoire, comprenant des moyens de comparaison d'une séquence desdites mesures avec deux, trois ou quatre séquences de données de référence représentatives chacune d'une manifestation physiologique ou comportementale dudit bovin ou dudit équidé appartenant au groupe comprenant :
- contractions musculaires de l'utérus ;
- coliques résultant de douleurs utérines ;
- levée de la queue ;
- suractivité ;
- des moyens de génération d'un signal de radiocommunication configurés de sorte à générer ledit signal dans le cas où ladite occurrence est détectée ;
- des moyens d'émission dudit signal généré à destination d'un collecteur formant relai ou d'un terminal.

2. Dispositif de détection et de signalisation selon la revendication 1, **caractérisé en ce que** ledit signal de radiocommunication est un signal numérique.

3. Dispositif de détection et de signalisation selon la revendication 2, **caractérisé en ce qu'**un identifiant dudit dispositif est encapsulé dans ledit signal numérique.

4. Dispositif de détection et de signalisation selon la revendication 1, **caractérisé en ce que** ladite séquence de données de référence présente, au moins pour une de ses composantes, au moins 3 pics, préférentiellement de 5 à 10 pics, espacés d'une durée de 3 à 4 secondes.

5. Dispositif de détection et de signalisation selon l'une quelconque des revendications 1 et 4, **caractérisé en ce que** lesdits moyens de traitement comprennent des moyens de détermination des coefficients de corrélation entre ladite séquence de mesures et lesdites séquences de référence.

6. Dispositif de détection et de signalisation selon la revendication 5, **caractérisé en ce que** lesdits moyens de traitement comprennent des moyens de comparaison d'un produit desdits coefficients de corrélation affectés d'une pondération avec une valeur seuil prédéterminée, et des moyens de génération d'une données d'alerte signifiant qu'une occurrence de ladite phase préparatoire a été détectée lorsque le résultat de ladite comparaison indique que ledit produit desdits coefficients de corrélation est supérieur ou égale à ladite valeur seuil prédéterminée.

7. Dispositif de détection et de signalisation selon la revendication 6, **caractérisé en ce que** la pondération de chacun desdits coefficients de corrélation dans ledit produit est égale à 1.

8. Dispositif de détection et de signalisation selon l'une quelconque des revendications 1 à 7, **caractérisé en ce qu'**il comprend un dispositif d'alimentation en énergie électrique autonome desdits moyens de mesure et/ou desdits moyens de traitement et/ou desdits moyens de génération d'un signal et/ou desdits moyens d'émission.

9. Dispositif de détection et de signalisation selon l'une quelconque des revendications 1 à 8, **caractérisé en ce qu'**il comprend un boitier présentant une portion inférieure configurée pour épouser la forme de ladite queue.

10. Dispositif de détection et de signalisation selon l'une quelconque des revendications 1 à 9, **caractérisé en ce que** ledit signal émis par lesdits moyens d'émission a une portée comprise entre 150 et 500 mètres.

11. Procédé de détection de la phase préparatoire de mise-bas d'un bovin ou d'un équidé, **caractérisé en ce qu'**il comprend les étapes suivantes :
- mesure de données représentatives des composantes de l'accélération d'une portion de la queue dudit bovin ou dudit équidé selon au moins un axe, et préférentiellement selon trois axes orthogonaux ;
- traitement des mesures desdites composantes destiné à détecter une occurrence de ladite phase préparatoire, comprenant une étape de comparaison d'une séquence desdites mesures avec deux, trois ou quatre séquences de données de référence représentatives chacune d'une manifestation physiologique ou comportementale dudit bovin ou dudit équidé appartenant au groupe comprenant :
- contractions musculaires de l'utérus ;
- coliques résultant de douleurs utérines ;
- levée de la queue ;
- suractivité.

12. Procédé de détection de la phase préparatoire de mise-bas d'un bovin ou d'un équidé selon la revendication 11, **caractérisé en ce que** ladite étape de traitement comprend :
- une étape de détermination des coefficients de corrélation entre ladite séquence de mesures et lesdites séquences de référence ;
- une étape de comparaison d'un produit desdits coefficients de corrélation affectés d'une pondération avec une valeur seuil prédéterminée, de façon à pouvoir générer un signal d'alerte, destiné à avertir qu'une occurrence de ladite phase préparatoire a été détectée, lorsque le résultat de ladite comparaison indique que ledit produit desdits coefficients de corrélation affectés d'une pondération est supérieur ou égale à ladite valeur seuil prédéterminée

13. Produit programme d'ordinateur téléchargeable depuis un réseau de communication et/ou enregistré sur un support lisible par ordinateur et/ou exécutable par un processeur, **caractérisé en ce qu'**il comprend des instructions de code de programme pour la mise en oeuvre de l'étape de traitement des mesures du procédé de détection selon l'une quelconque des revendications 11 et 12, lorsque ledit programme est exécuté par un un dispositif de détection et de signalisation de la phase préparatoire de mise-bas d'un bovin ou d'un équidé selon l'une quelconque des revendications 1 à 10.

## Patentansprüche

1. Vorrichtung zum Erkennen und Anzeigen der Vorbereitungsphase der Niederkunft bei Rindern oder Pferden, die geeignet ist, am Schwanz der Rinder oder Pferde befestigt zu werden, umfassend:
- Mittel zum Messen der Komponenten der Beschleunigung der Vorrichtung entlang mindestens einer Achse und vorzugsweise entlang dreier orthogonaler Achsen;
- Mittel zur Bearbeitung der Messungen der Komponenten, die dazu bestimmt sind, ein Vorliegen der Vorbereitungsphase zu erkennen, umfassend Mittel zum Vergleichen einer Sequenz der Messungen mit zwei, drei oder vier Referenzdatensequenzen, die jeweils für eine physiologische oder verhaltensmäßige Manifestierung des Rindes oder Pferdes repräsentativ sind, die der Gruppe angehört, umfassend:
- Muskelkontraktionen des Uterus;
- Koliken, die von Uterusschmerzen stammen;
- Anheben des Schwanzes;
- Überaktivität;
- Mittel zur Erzeugung eines Funkkommunikationssignals, die eingerichtet sind, um das Signal zu erzeugen, wenn das Vorliegen erkannt wird;
- Mittel zum Senden des erzeugten Signals in Richtung eines Kollektors, der ein Relais oder ein Endgerät bildet.

2. Vorrichtung zum Erkennen und Anzeigen nach Anspruch 1, **dadurch gekennzeichnet, dass** das Funkkommunikationssignal ein digitales Signal ist.

3. Vorrichtung zum Erkennen und Anzeigen nach Anspruch 2, **dadurch gekennzeichnet, dass** ein Identifikator der Vorrichtung in dem digitalen Signal verkapselt ist.

4. Vorrichtung zum Erkennen und Anzeigen nach Anspruch 1, **dadurch gekennzeichnet, dass** die Referenzdatensequenz zumindest für eine ihrer Komponenten mindestens 3 Peaks, vorzugsweise 5 bis 10 Peaks, im Abstand von 3 bis 4 Sekunden aufweist.

5. Vorrichtung zum Erkennen und Anzeigen nach einem der Ansprüche 1 und 4, **dadurch gekennzeichnet, dass** die Bearbeitungsmittel Mittel zur Bestimmung der Korrelationskoeffizienten zwischen der Sequenz von Messungen und den Referenzsequenzen umfassen.

6. Vorrichtung zum Erkennen und Anzeigen nach Anspruch 5, **dadurch gekennzeichnet, dass** die Bearbeitungsmittel Mittel zum Vergleichen eines Produkts der Korrelationskoeffizienten, denen eine Gewichtung zugeordnet ist, mit einem vorbestimmten Grenzwert und Mittel zur Erzeugung eines Alarmdatums umfassen, das anzeigt, dass ein Vorliegen der Vorbereitungsphase erkannt wurde, wenn das Resultat des Vergleichs anzeigt, dass das Produkt der Korrelationskoeffizienten größer oder gleich dem vorbestimmten Grenzwert ist.

7. Vorrichtung zum Erkennen und Anzeigen nach Anspruch 6, **dadurch gekennzeichnet, dass** die Gewichtung jedes der Korrelationskoeffizienten in dem Produkt gleich 1 ist.

8. Vorrichtung zum Erkennen und Anzeigen nach einem der Ansprüche 1 bis 7, **dadurch gekennzeichnet, dass** sie eine autonome Vorrichtung zur Stromversorgung der Messmittel und/oder der Bearbeitungsmittel und/oder der Signalerzeugungsmittel und/oder der Sendemittel umfasst.

9. Vorrichtung zum Erkennen und Anzeigen nach einem der Ansprüche 1 bis 8, **dadurch gekennzeichnet, dass** sie ein Gehäuse umfasst, das einen unteren Abschnitt aufweist, der eingerichtet ist, um sich an die Form des Schwanzes anzupassen.

10. Vorrichtung zum Erkennen und Anzeigen nach einem der Ansprüche 1 bis 9, **dadurch gekennzeichnet, dass** das von den Sendemitteln gesandte Signal eine Reichweite zwischen 150 und 500 Meter hat.

11. Verfahren zum Erkennen der Vorbereitungsphase der Niederkunft bei Rindern oder Pferden, **dadurch gekennzeichnet, dass** es die folgenden Schritte umfasst:
- Messen von Daten, die für die Komponenten der Beschleunigung eines Abschnitts des Schwanzes des Rindes oder Pferdes entlang mindestens einer Achse und vorzugsweise entlang dreier orthogonaler Achsen repräsentativ sind;
- Bearbeitung der Messungen der Komponenten, die dazu bestimmt ist, ein Vorliegen der Vorbereitungsphase zu erkennen, umfassend einen Schritt des Vergleichens einer Sequenz der Messungen mit zwei, drei oder vier Referenzdatensequenzen, die jeweils für eine physiologische oder verhaltensmäßige Manifestierung des Rindes oder Pferdes repräsentativ sind, die der Gruppe angehört, umfassend:
- Muskelkontraktionen des Uterus;
- Koliken, die von Uterusschmerzen stammen;
- Anheben des Schwanzes;
- Überaktivität.

12. Verfahren zum Erkennen der Vorbereitungsphase der Niederkunft bei Rindern oder Pferden nach Anspruch 11, **dadurch gekennzeichnet, dass** der Bearbeitungsschritt umfasst:
- einen Schritt der Bestimmung der Korrelationskoeffizienten zwischen der Sequenz von Messungen und den Referenzsequenzen;
- einen Schritt des Vergleichens eines Produkts der Korrelationskoeffizienten, denen eine Gewichtung zugeordnet ist, mit einem vorbestimmten Grenzwert, um ein Alarmsignal erzeugen zu können, das dazu bestimmt ist anzuzeigen, dass ein Vorliegen der Vorbereitungsphase erkannt wurde, wenn das Resultat des Vergleichs anzeigt, dass das Produkt der Korrelationskoeffizienten, denen eine Gewichtung zugeordnet ist, größer oder gleich dem vorbestimmten Grenzwert ist.

13. Computerprogrammprodukt, das von einem Kommunikationsnetz fernladbar und/oder auf einem von einem Computer lesbaren Träger aufgezeichnet und/oder von einem Prozessor ausführbar ist, **dadurch gekennzeichnet, dass** es Programmcodebefehle für den Einsatz des Bearbeitungsschrittes der Messungen des Erkennungsverfahrens nach einem der Ansprüche 11 und 12 umfasst, wenn das Programm von einer Vorrichtung zum Erkennen und Anzeigen der Vorbereitungsphase der Niederkunft bei Rindern oder Pferden nach einem der Ansprüche 1 bis 10 ausgeführt wird.

## Claims

1. A device for detecting and notifying the preparatory phase of parturition of a bovine or equine animal, adapted to be attached to the tail of said bovine or said equine, comprising:
- means for measuring the components of the acceleration of said device along at least one axis, and preferably along three orthogonal axes;
- means for processing the measurement data of said components, intended for detecting an occurrence of said preparatory phase, comprising means for comparing a sequence of said measurement data with two, three or four reference data sequences, each representative of a physiological or behavioural manifestation of said bovine or said equine belonging to the group comprising:
- muscle contractions of the womb;
- colics resulting from uterine pain;
- tail rising;
- overactivity;
- means for generating a radiocommunication signal configured so as to generate said signal in case where said occurrence is detected;
- means for transmitting said generated signal to a collector forming a relay or to a terminal.

2. The device for detecting and notifying according to claim 1, wherein said radiocommunication signal is a digital signal.

3. The device for detecting and notifying according to claim 2, wherein an identifier of said device is encapsulated in said digital signal.

4. The device for detecting and notifying according to claim 1, wherein said reference data sequence has, at least for one of its components, at least 3 peaks, preferably 5 at 10 peaks, 3 to 4 seconds spaced apart.

5. The device for detecting and notifying according to any of the claims 1 and 4, **characterized in that** said processing means include means for determining correlation coefficients between said sequence of readings and said reference sequences.

6. The device for detecting and notifying according to claim 5, wherein said processing means comprise means for comparing a weighted product of said correlation coefficients with a predetermined threshold value, and means for generating warning data, meaning that an occurrence of said preparatory phase has been detected when the result of the comparison indicates that said product of said correlation coefficients is greater than or equal to said predetermined threshold value.

7. The device for detecting and notifying according to claim 6, wherein the weighting of each of said correlation coefficients is said product is 1.

8. The device for detecting and notifying according to any of the claims 1 to 7, **characterized in** this it comprises an autonomous electric power supply device for said measuring means and/or said processing means and/or said means for generating a signal and/or said transmitting means.

9. The device for detecting and notifying according to any of the claims 1 to 8, **characterized in that** it comprises a housing having a lower portion configured to match the shape of said tail.

10. A device for detecting and notifying according to any of the claims 1 to 9, wherein said signal transmitted by said transmitting means has a range between 150 and 500 meters.

11. A method for detecting the preparatory phase of parturition of a bovine or equine animal, **characterised in that** it comprises the following steps:
- measuring data representative of the components of the acceleration of a portion of the tail of said bovine or equine along at least one axis, and preferably along three orthogonal axes;
- processing the measurement data of said components, intended for detecting an occurrence of said preparatory phase, comprising a step of comparing a sequence of said measurement data with two, three or four reference data sequences, each representative of a physiological or behavioural manifestation of said bovine or said equine belonging to the group comprising:
- muscle contractions of the womb;
- colics resulting from uterine pain;
- tail rising;
- overactivity;

12. The method for detecting the preparatory phase of parturition of a bovine or equine animal according to claim 11, **characterized in that** said processing step comprises:
- a step of determining the correlation coefficients between said sequence of measurement data and said reference sequences;
- a step of comparing a weighted product of said correlation coefficients with a predetermined threshold value, so as to generate a warning signal, to warn that an occurrence of said preparatory phase has been detected when the result of said comparison indicates that said product of said weighted correlation coefficients is greater than or equal to said predetermined threshold value.

13. A computer program product, which is downloadable from a communication network and/or recorded on a computer-readable medium and/or executable by a processor, **characterised in that** it comprises the program code instructions for implementing the step for processing the measurement data of the detection method according to any of the claims 11 and 12, when said program is executed by a device for detecting and notifying the preparatory phase of parturition of a bovine or equine according to any one of claims 1 to 10.
